# EUROPEAN PATENT SPECIFICATION

(11) **EP 2 264 159 B1**
(45) Date of publication and mention of the grant of the patent: **14.05.2014**
(21) Application number: 10182311.0
(22) Date of filing: 26.11.2007
(51) Int. Cl.: C12N 9/20, C12P 7/40

(54) **Lipolytic enzyme variants**
Lipolytische enzymvarianten
Variants d'enzyme lipolytique

(30) Priority: 28.11.2006 DK 200601560
(43) Date of publication of application: 22.12.2010
(62) Divisional of application: 07847324.6
(73) Proprietor: Novozymes A/S, 2880 Bagsvaerd (DK)
(72) Inventor: De Maria, Leonardo, 2000 Frederiksberg (DK); Brask, Jesper, 2880 Bagsværd (DK); Skjøt, Michael, 4040 Jyllinge (DK); Patkar, Shamkant Anant, 2800 Lyngby (DK); Borch, Kim, 3460 Birkerød (DK); Svendsen, Allan, 2970 Hørsholm (DK)

(56) References cited:
- WO-A-2004/024954
- LUTZ S: "Engineering lipase B from Candida antarctica", TETRAHEDRON ASYMMETRY 20 SEP 2004 UNITED KINGDOM, vol. 15, no. 18, 20 September 2004 (2004-09-20), pages 2743-2748, XP002434915, ISSN: 0957-4166
- ROTTICCI D ET AL: "Improved enantioselectivity of a lipase by rational protein engineering", CHEMBIOCHEM - A EUROPEAN JOURNAL OF CHEMICAL BIOLOGY, WILEY VCH, WEINHEIM, DE, vol. 2, no. 10, 1 October 2001 (2001-10-01), pages 766-770, XP002475602, ISSN: 1439-4227, DOI: DOI:10.1002/1439-7633(20011001)2:10<766::A ID-CBIC766>3.0.CO;2-K
- ZHANG NINGYAN ET AL: "Improving tolerance of Candida antarctica lipase B towards irreversible thermal inactivation through directed evolution", PROTEIN ENGINEERING, OXFORD UNIVERSITY PRESS, SURREY, GB, vol. 16, no. 8, 1 August 2003 (2003-08-01) , pages 599-605, XP002404458, ISSN: 0269-2139
- MAGNUSSON ANDERS O ET AL: "Creating space for large secondary alcohols by rational redesign of Candida antarctica lipase B.", CHEMBIOCHEM : A EUROPEAN JOURNAL OF CHEMICAL BIOLOGY JUN 2005, vol. 6, no. 6, June 2005 (2005-06), pages 1051-1056, XP002434917, ISSN: 1439-4227
- PATKAR S ET AL: "Effect of mutations in Candida antarctica B lipase", CHEMISTRY AND PHYSICS OF LIPIDS, LIMERICK, IR, vol. 93, no. 1-2, 1 June 1998 (1998-06-01) , pages 95-101, XP002994699, ISSN: 0009-3084, DOI: DOI:10.1016/S0009-3084(98)00032-2
- OTTOSSON J ET AL: "Rational design of enantioselective enzymes requires considerations of entropy.", PROTEIN SCIENCE : A PUBLICATION OF THE PROTEIN SOCIETY SEP 2001, vol. 10, no. 9, September 2001 (2001-09), pages 1769-1774, XP002434918, ISSN: 0961-8368
- QIAN ZHEN ET AL: "Improving the catalytic activity of Candida antarctica lipase B by circular permutation.", JOURNAL OF THE AMERICAN CHEMICAL SOCIETY 5 OCT 2005, vol. 127, no. 39, 5 October 2005 (2005-10-05), pages 13466-13467, XP002434916, ISSN: 0002-7863
- SUEN WEN-CHEN ET AL: "Improved activity and thermostability of Candida antarctica lipase B by DNA family shuffling", 1 February 2004 (2004-02-01), PROTEIN ENGINEERING, DESIGN AND SELECTION, OXFORD JOURNAL, LONDON, GB, PAGE(S) 133 - 140, XP002470262, ISSN: 1741-0126 * the whole document *
- UPPENBERG J ET AL: "The sequence, crystal structure determination and refinement of two crystal forms of lipase B from Candida antarctica", 15 April 1994 (1994-04-15), STRUCTURE, CURRENT BIOLOGY LTD., PHILADELPHIA, PA, US, PAGE(S) 293 - 308, XP002354898, ISSN: 0969-2126 * the whole document *

## Description

### FIELD OF THE INVENTION

The present invention relates to a polypeptide with lipolytic enzyme activity and to a method of preparing it.

### BACKGROUND OF THE INVENTION

WO8802775 describes *Candida antarctica* lipase B (CALB). Uppenberg, Hansen, Patkar, Jones, Structure 2, 293-308 (1994) describe the amino acid sequence and three-dimensional (3D) structure of CALB. The 3D structure can be found in the Research Collaboratory for Structural Bioinformatics Protein Data Bank (RCBS PDB) (http://www.rcsb.org/), its identifier being 1TCA.

CALB variants are described in Zhang et al. Prot.Eng. 2003, 16, 599-605; Lutz. 2004, Tetrahedron: Asymmetry, 15, 2743-2748; Qian and Lutz, JACS, 2005, 127, 13466-13467; and in WO 2004/024954; Roticci et al, Chembiochem 2001,2, 766 - 770; Magnussen et al, Chembiochem 2005, 6, 1051-1056; Patkar et al, 1998, 93, 95-101.

WO9324619 describes a lipase from *Hyphozyma sp.* Amino acid sequences for other lipases can be found in UniProt [the Universal Protein Resource] with accession numbers Q4pep1, Q7RYD2, Q2UE03, Q4WG73, Q6BVP4 and Q4HUY1.

### SUMMARY OF THE INVENTION

The inventors performed molecular dynamics (MD) simulation on the 1TCA structure. The analysis reveals two hitherto unknown lids with a marked mobility, Lid 1 consisting of residues from 135 or 136 to 155 or 160, and Lid 2 consisting of residues 267-295. The simulation indicated a more closed like form in water solution and a more fully open form in organic solvent solution. The analysis revealed important areas in the 3D structure for affecting the activity and functionality of the lipase, and the inventors used this to design lipolytic enzyme variants with increased specific activity, particularly towards bulky substrates (e.g. esters of a branched acid or long-chain fatty acid and/or a secondary alcohol) and/or increased activity at high pH (higher pH optimum) and/or increased enantioselectivity.

Further, the inventors have selected amino acid residues and designed lipolytic enzyme variants based on an alignment of CALB with some homologous lipase sequences.

Disclosed is a method of preparing a polypeptide, comprising
a) selecting a parent polypeptide which has lipolytic enzyme activity and has an amino acid sequence with at least 30 % identity to CALB (SEQ ID NO: 1),
b) selecting one or more amino acid residues in the sequence corresponding to any of residues 1, 13, 25, 38-51, 53-55, 58, 69-79, 91, 92, 96, 97, 99, 103, 104-110, 113, 132-168, 173, 187-193, 197-205, 215, 223-231, 242, 244, 256, 259, 261-298, 303, 305, 308-313, or 315 of CALB (SEQ ID NO: 1),
c) altering the selected amino acid sequence wherein the alteration comprises substitution or deletion of the selected residue(s) or insertion of at least one residue adjacent to the selected residue(s),
d) preparing an altered polypeptide having the altered amino acid sequence,
e) determining the lipolytic enzyme activity or enantioselectivity towards carboxylic ester bonds of the altered polypeptide, and
f) selecting an altered polypeptide which has higher lipolytic enzyme activity or a higher enantioselectivity than the parent polypeptide.

The invention provides a polypeptide which:
a) has lipolytic enzyme activity, and
b) has an amino acid sequence which has at least 80 % identity (particularly at least 90 % or at least 95 % identity) to CALB (SEQ ID NO: 1) and comprises an amino acid substitution, corresponding to D223G when compared to SEQ ID NO: 1.

Finally, the invention provides use of the above variant polypeptide in a lipase-catalyzed process.

### BRIEF DESCRIPTION OF THE DRAWINGS

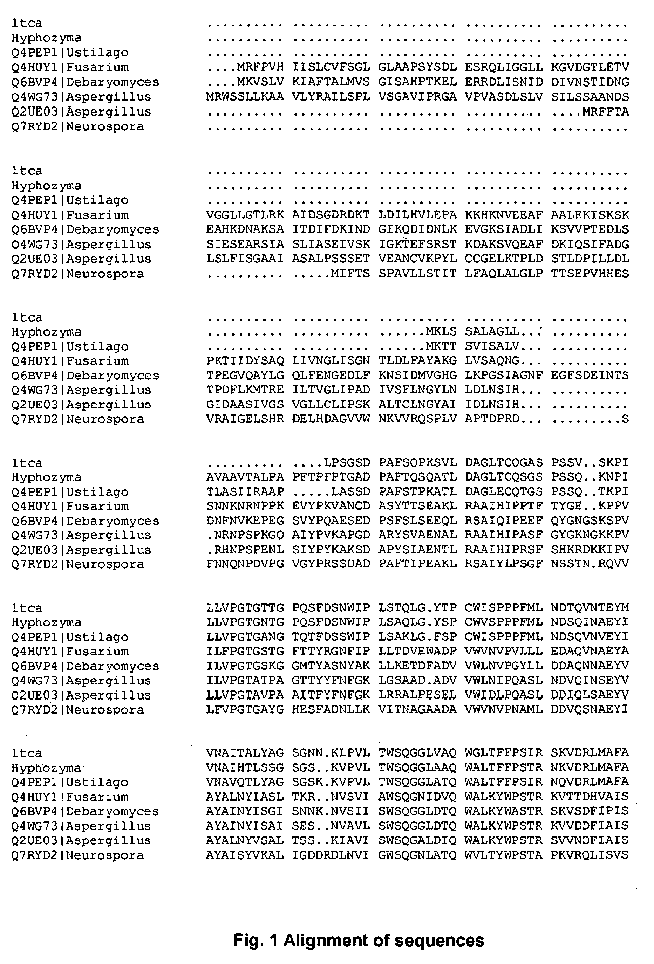

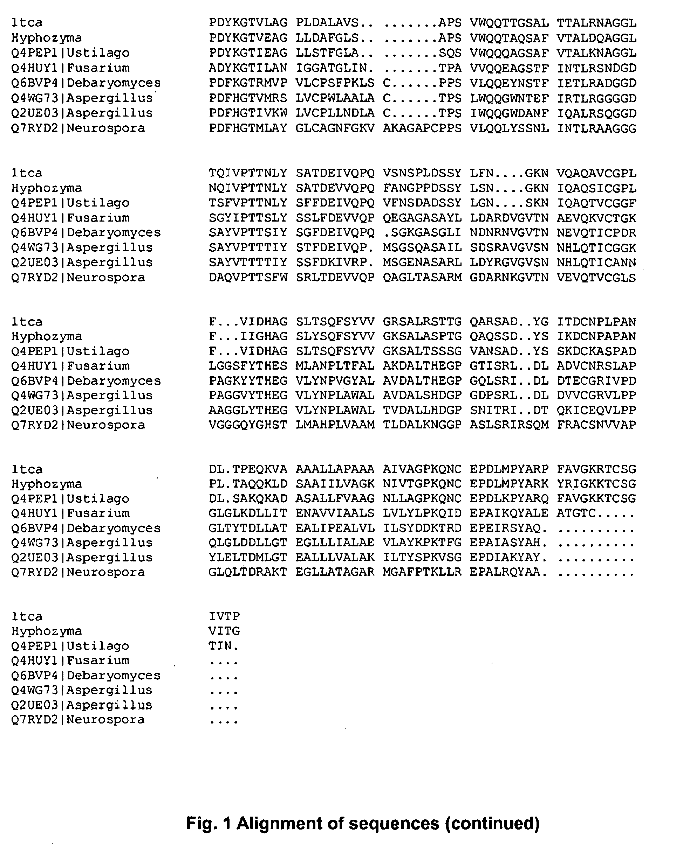

Fig. 1 shows an alignment of amino acid sequences SEQ ID NOS. 1-7.

### DETAILED DESCRIPTION OF THE INVENTION

### Parent polypeptide

The parent polypeptide which can be used in the disclosed method has lipolytic enzyme activity and has an amino acid sequence with at least 30 % identity (particularly at least 40 %, at least 50 %, at least 60 %, at least 70 %, at least 80 % or at least 90 %) to *Candida antarctica* lipase B (CALB, SEQ ID NO: 1) which is described in WO8802775, and whose sequence is given in Uppenberg, J., Hansen, M.T., Patkar, S., Jones, T.A., Structure v2 pp. 293-308, 1994. The parent polypeptide may be any of the following lipases. An alignment is shown in Fig. 1.
SEQ ID NO: 1: *Candida antarctica* lipase B (CALB), 1TCA
SEQ ID NO: 2: *Hyphozyma* sp., WO9324619
SEQ ID NO: 3: *Ustilago maydis*, UniProt Q4pep1
SEQ ID NO: 4: *Gibberella zeae (Fusarium graminearum)*, UniProt Q4HUY1
SEQ ID NO: 5: *Debaryomyces hansenii*, UniProt Q6BVP4
SEQ ID NO: 6: *Aspergillus fumigatus*, UniProt Q4WG73
SEQ ID NO: 7: *Aspergillus oryzae*, UniProt Q2UE03
SEQ ID NO: 8: *Neurospora crassa* lipase, UniProt Q7RYD2

The alignment was done using the needle program from the EMBOSS package (http://www.emboss.org) version 2.8.0 with the following parameters: Gap opening penalty: 10.00, Gap extension penalty: 0.50, Substitution matrix: EBLOSUM62. The software is described in EMBOSS: The European Molecular Biology Open Software Suite (2000), Rice,P. Longden,I. and Bleasby,A., Trends in Genetics 16, (6) pp276-277. The program needle implements the global alignment algorithm described in Needleman, S. B. and Wunsch, C. D. (1970) J. Mol. Biol. 48, 443-453, and Kruskal, J. B. (1983).

Other parent polypeptides may aligned to the sequences in Fig. 1 by the same method or by the methods described in D. Sankoff and J. B. Kruskal, (ed.), Time warps, string edits and macromolecules: the theory and practice of sequence comparison, pp. 1-44 Addison Wesley.

### Three-dimensional (3D) structure and lids

In the 3D structure 1TCA, the inventors identified two lids with high mobility at amino acid residues from 135 or 136 to 155 or 160 (Lid 1) and residues 267-295 (Lid 2) of SEQ ID NO: 1. The MD simulation indicated that the following regions are of particular interest because of a particularly high mobility: residues 141-149 in Lid 1 and the following regions in Lid 2: 267-269, 272, 275-276, 279-280, 282-283, 286-290.

### Selection of amino acid residue

According to the disclosed method an amino acid residue may be selected having a non-hydrogen atom within 8 A of a non-hydrogen atom of a residue in Lid 1 or Lid 2 in a 3D structure. This criterion selects the following residues in the structure 1TCA: 38-51, 53-55, 58, 69-79, 104-110, 113, 132-168, 173, 187-193, 197-205, 223-231, 259, 261-298, 305, 308-313, 315 of SEQ ID NO: 1.

The residue may particularly be selected within 6 A of the lids, leading to the following residues in 1TCA: 40-42, 46-51, 54, 58, 70-77, 79, 104-107, 109, 133-165, 167, 173, 187-192, 197-203, 223-225, 228-229, 261-297, 308-312.

An amino acid residue may also be selected by aligning homologous lipolytic enzyme sequences and selecting a residue at a position with variability, i.e. a position where different sequences have different residues. Thus, the following residues in CALB (SEQ ID NO: 1) can be selected by a comparison with *Hyphozyma* lipase (SEQ ID NO: 2) based on the alignment shown in Fig. 1: 1, 3, 5, 10, 12-15, 25, 30, 31, 32, 57, 62, 66, 76, 78, 80, 83, 88, 89, 91, 92, 96, 97, 114, 121, 123, 143, 147-149, 159, 163, 164, 168, 169, 174, 188, 194, 195, 197, 199, 205, 210, 214, 215, 221, 223, 229, 238, 242, 244, 249, 251, 254, 256, 261, 265, 268, 269, 272-274, 277-280, 282-284, 287, 303-306, 309, 314, 315, 317.

The following residues are of special interest: 1, 13, 25, 38, 42, 74, 140, 143, 147, 164, 168, 190, 199, 215, 223, 242, 244, 256, 265, 277, 280, 281, 283, 284, 285, 292, 303, 315 of CALB (SEO ID NO: 1).

Corresponding residues in other lipases may be identified from a sequence alignment. An alignment of several sequences is shown in Fig. 1. Other sequences may be aligned by known methods, such as AlignX (a component of vector nti suite 9.0.0) using standard settings.

### Altered amino acid sequence

The altered amino acid sequence is derived from the parent sequence by making an amino acid alteration at one or more selected positions, and optionally also at other positions. Each amino acid alteration consists of substitution or deletion of the selected residue or insertion of at least one residue adjacent to the selected residue at the N- or C-terminal side.

### Particular substitutions

The following alterations in SEQ ID NO: 1 may optionally be combined:
- K13Q, A25G, P38V,L,S, T42N, N74Q, V781, Y91S, A92S, N96S, L99V, W104H, D134L,M,N, T138L, L140E, P143S,L, D145S, A146T, L147N,F, A148P, V149P, S150A, W155Q,N, Q157N, T158S, L163F, T164V, R168D, V1901,A, S197L,G, L199P, V2151, D223G, T229Y, R242A, T244P, T256K, L261A, D265P, P268A, E269Q, L277I, P280V, A281 S, A283K, A284N, 1285E,D, G288D, N292C,Q, P303K, K308D, ot V3151.
- Multiple substitutions:
   ○ I258D G288D
   ○ S197G L199P
   ○ T164V L163F
   ○ V190X Q157X
   ○ A281X W155X
   ○ D223X A281 X
   ○ D223X I285X
   ○ A281X I285X
   ○ A281X W155X A148X
   ○ D145X K308X K138X
   ○ D223X A281X I285X
- Insertions: L147FN, G137ASV, V190GAH, L1QL, L1QGPL
- Deletion: N97*

Based on an alignment such as that shown in Fig. 1, one sequence may be used as a template for alterations in another sequence. Thus, Lid 1 or Lid 2 of one sequence may be substituted with the corresponding lid region of another sequence. The following variants are designed by altering Lid 1 of CALB using the indicated polypeptide as template:
- Q7RYD2 (*Neurospora crassa*) as termplate: Y135F K136H V139M G142Y P143G D145C L147G A148N V149F S150GKVAKAGAPC A151 P W155L
- Q4HUY1 (*Fusarium graminearum*) as template: V1391 G142N P1431 L144G D145G L147T A148G V149L S150IN A151T S153A W155V
- *Hyphozyma sp.* lipase as template: L140E P143L L147F A148G V149L.
- Q4PEP1 (*Ustilago maydis*) as template: V139I L140E P143L D145S A146T L147F A148G V149L S150A A151S P152Q.

Each of the above variants may optionally be combined with N292C and/or D223G and/or A281 S and/or 1285E.

The following substitutions may be made in SEQ ID NO: 2 (*Hyphozyma sp.* lipase): V1921, Q159N, D136L,M,N, P41V,L, S50A, N45S, W106H.

### Nomenclature for amino acid alterations

In this specification, an amino acid substitution is described by use of one-letter codes, e.g. W155Q. X is used to indicate a substitution with any different residue (e.g. V190X). Multiple substitutions are concatenated, e.g. S197G L199P to indicate a variant with two substitutions. Alternatives are indicated by commas, e.g. W155Q,N to indicate a substitution of W155 with Q or N. An asterisk indicates a deletion. An insertion is indicated as substitution of one residue with two or more residues (e.g. L147FN)

### Lipolytic enzyme activity

The parent and the variant polypeptides have lipolytic enzyme activity (particularly lipase activity), i.e. they are able to hydrolyze carboxylic ester bonds to release carboxylate (EC 3.1.1), particularly ester bonds in triglycerides (triacylglycerol lipase activity, EC 3.1.1.3).

The enzyme activity may be expressed as specific activity, i.e. hydrolytic activity per mg of enzyme protein. The amount of enzyme protein can be determined e.g. from absorption at 280 nm or by active-site titration (AST), as described by Rotticci et al. Biochim. Biophys. Acta 2000, 1483, 132-140.

### Enantioselectivity

Enantioselectivity is often an important parameter in CaLB catalyzed reactions, both in the hydroysis and in the synthesis direction. The substrate can be a racemic mixture of two enantiomers, or it can be a prochiral meso form. In both cases a single enantiomer product is often desired. Enantiomeric excess (ee) is measured by quantifying the amount of both product enantiomers, and then calculating ee = (yield of desired enantiomer - yield of other enantiomer) / (sum of both yields). The quantification is often by chiral gas chromatography (GC) or high-performance liquid chromatography (HPLC).

### Use of lipolytic enzyme variant

The lipolytic enzyme variant may be used for biocatalysis in a lipase-catalyzed reaction, both in ester hydrolysis and synthesis reactions, e.g. in synthesis of some polymers. The lipase-catalyzed reaction may be:
a) hydrolysis with a carboxylic acid ester and water as reactants, and a free carboxylic acid and an alcohol as products,
b) ester synthesis with a free carboxylic acid and an alcohol as reactants, and a carboxylic acid ester as product,
c) alcoholysis with a carboxylic acid ester and an alcohol as reactants, or
d) acidolysis with a carboxylic acid ester and a free fatty acid as reactants.

Like CALB, the variant of the invention may particularly be used in applications where the enzyme's chemo-, regio-, and/or stereoselectivity, stability and reaction rate or the ability to accept a relatively broad range of substrates is important. The reaction products are typically used in the chemical, fine chemical, pharmaceutical, or agrochemical industry, or as food ingredients. The variant may be immobilized, e.g. by adsorption on an adsorbent resin such as polypropylene.

The ester in the lipase-catalyzed reaction may have a bulky acid group or a bulky or secondary alcohol part, such as pNP 2-Me-butyrate, 6,8-difluro-4-methylumbelliferyl octanoate (DiFMU octanoate) or an iso-propyl fatty acid ester (e.g. C₁₆-C₁₈ fatty acid which may be saturated or unsaturated).

The variant may be used as described for CALB in A. J. J. Straathof, S. Panke, A. Schmid. Curr. Opin. Biotechnol. 2002, 13, 548-556; E. M. Anderson, K. M. Larsson, O. Kirk. Biocat. Biotrans. 1998, 16, 181-204; R. A. Gross, A. Kumar, B. Kaira. Chem. Rev. 2001, 101, 2097-2124).

### EXAMPLES

### Example 1: Selection of amino acid residues by molecular dynamics

From Molecular Dynamics simulations 2 regions were found to be of high importance for the activity of *Candida antarctica* lipase B, as follows.

CHARMm was used to prepare the 1TCA structure for the simulations. Hydrogen atoms were added to both protein and waters using the command HBUILD. The system was embedded in explicit water molecules and confined to a cubic box of side equal to 90 Angstroms. There were in total 24630 water molecules including those already present in the 1TCA structure. A simulation at constant temperature, 300K, and constant pressure, 1.01325 atmospheres, was performed for a total of 20 nanoseconds using NAMD. Berendsen's coupling method was used to keep the temperature and the pressure at the desired values. The results of the simulation were then analyzed using CHARMm (References for CHARMM: MacKerell, A. D., Bashford, D., Bellott, M., Dunbrack, R. L., Evanseck, J. D., Field, M. J., Fischer, S., Gao, J., Guo, H., Ha, S., Joseph-McCarthy, D., Kuchnir, L., Kuczera, K., Lau, F. T. K., Mattos, C., Michnick, S., Ngo, T., Nguyen, D. T., Prodhom, B., Reiher, W. E., Roux, B., Schlenkrich, M., Smith, J. C., Stote, R., Straub, J., Watanabe, M., Wiorkiewicz-Kuczera, J., Yin, D., Karplus, M. J. Phys. Chem. B 1998, 102, 3586; MacKerell, A. D., Jr., Brooks, B., Brooks, C. L., III, Nilsson, L., Roux, B., Won, Y., Karplus, M. In The Encyclopedia of Computational Chemistry; Schleyer, P. v. R. et al., Eds.; John Wiley & Sons: Chichester, 1998; Vol. 1, p 271; Brooks, B. R., Bruccoleri, R. E., Olafson, B. D., States, D. J., Swaminathan, S., Karplus, M. J. Comput. Chem. 1983, 4, 187).

The analysis revealed hitherto unknown lids with high mobility. Several regions were found to move when the enzyme is in solution. It was concluded that the enzyme functionality and specificity are dependent on this mobility and the specific structure present in the media of choice for the hydrolysis or the synthesis reaction. The simulation indicated a more closed like form in water solution and a more fully open form in organic solvent solution, i.e. more like the crystal structure in some surfactant containing water solution.

Using calculation of the isotropic Root Mean Square Displacements for the C-alpha atoms of the residues in CALB along the above mentioned simulation, regions with increased mobility were identified. The mobile lid regions were found to be residues 136-160 for Lid1 and residues 267-295 for Lid2. It was concluded that the residues in the neighborhood of these novel lids interact with the lid mobility and are thus very important for the activity of the enzyme.

### Example 2: Hydrolysis reactions

Hydrolytic activity of the variants was evaluated on pNP-butyrate, racemic pNP 2-methylbutyrate, and 6,8-difluro-4-methylumbelliferyl octanoate (DiFMU octanoate). Racemic pNP 2-Me-butyrate was synthesized according to J. Biol. Chem. 1971, 246, 6019-6023. DiFMU octanoate, purchased from Molecular Probes, has previously been reported by Lutz et al. (J. Am. Chem. Soc. 2005, 127, 13466-13467) in CALB assays. Whereas pNP 2-Me-butyrate selects variants with improved acceptance of substrates with a bulky acid group, DiFMU octanoate selects variants with improved acceptance of a bulky alcohol part. Reactions were performed in 50 mM aqueous phosphate buffer, pH 7.0 with 0.1% Triton X-100. Reaction kinetic was followed for approx. 15 min in microtiter plates, measuring at 405 nm (pNP) or 350/485 nm (ex/em for DiFMU). Activities were normalized based on enzyme A₂₈₀.

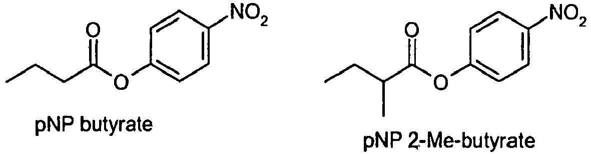

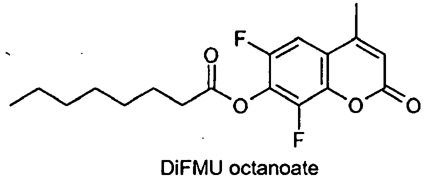

Results are shown below as activity for the various substrates in % of CALB wild-type.

| **Variant** | **pNP butyrate** | **pNP 2-Me-butyrate** | **DiFMU octanoate** |
|---|---|---|---|
| N74Q | 77 | 113 | 110 |
| P143S | 50 | 113 | 42 |
| A281S | 215 | 232 | 208 |
| P38S | 35 | 107 | 44 |
| N292Q | 73 | 158 | 105 |
| L1QGPL | 63 | 144 | 85 |
| L1QL | 65 | 193 | 49 |
| I285E | 233 | 332 | 236 |
| L147F | 98 | 232 | 90 |
| L147N | 79 | 178 | 79 |
| N292C | 80 | 282 | 90 |
| L140E | 51 | 151 | 79 |
| P143L | 79 | 192 | 112 |
| A146T | 55 | 126 | 42 |
| P280V | 48 | 100 | 36 |
| A283K | 104 | 115 | 94 |
| A284N | 65 | 125 | 19 |
| T103G, A148P | 70 | 167 | 0 |
| W104H, A148P | 11 | 146 | 0 |
| N74Q, A281 S | 88 | 156 | 0 |
| V190A | 64 | 143 | |
| L199P | 74 | 162 | 75 |
| T256K | 105 | 120 | 79 |
| T42N | 35 | 216 | 47 |
| R242A | 24 | 119 | 39 |
| V215I | 105 | 133 | 43 |
| T164V | 75 | 130 | 80 |
| L163F, T164V | 81 | 160 | 92 |
| D265P | 28 | 117 | 44 |
| P303K | 35 | 108 | 50 |
| R168D | 62 | 122 | 53 |
| A25G | 66 | 111 | 26 |
| V315I | 65 | 102 | 19 |
| T244P | 56 | 146 | 20 |
| K13Q | 56 | 122 | 39 |
| L277I | 53 | 137 | 51 |
| Y91 S, A92S, N96S, N97*, L99V | 39 | 135 | 76 |
| D223G | 830 | 3621 | 820 |
| Parent (CALB) | 100 | 100 | 100 |

The results demonstrate that the specific activity towards a bulky substrate (ester with a branched fatty acid) can be increased up to 37-fold by substituting a single selected amino acid residue.

### Example 3: Variants with lid replacement

Variants based on CaLB wild-type (SEQ ID NO: 1) were designed by replacing lid 1 with the corresponding residues of the *Fusarium* lipase (SEQ ID NO: 4), the *Debaryomyces* lipase (SEQ ID NO: 5) or the *Neurospora* lipase (SEQ ID NO: 8). Further variants were designed by combining this with a single substitution of a selected residue (A281 S). Results are expressed as activity in % of CALB activity on the same substrate.

| **Variant** | **pNP butyrate** | **pNP 2-Me-butyrate** | **DiFMU octanoate** |
|---|---|---|---|
| V1391, G142N, P143I, L144G, D145G, L147T, A148G, V149L, S150IN, A151T, S153A, W155V | 187 | 661 | 836 |
| Y135F, V139R, L140M, A141V, G142P, P143V, D145C, A146P, L147S, A148F, V149P, S150KLSC, A151P, W155L | 62 | 306 | 14 |
| Y135F, K136H, V139M, G142Y, P143G, D145C, L147G, A148N, V149F, S150GKVAKAGAPC, A151P, W155L | 341 | 1223 | 14 |
| V139I, G142N, P143I, L144G, D145G, L147T, A148G, V149L, S150IN, A151T, S153A, W155V, A281S | 1052 | 1612 | 631 |
| Y135F, K136H, V139M, G142Y, P143G, D145C, L147G, A148N, V149F, S150GKVAKAGAPC, A151P, W155L, A281S | 378 | 2397 | 76 |

The results demonstrate that the specific activity towards a bulky substrate can be significantly increased by replacing the lid of one lipase with the lid of another lipase, and that this can be further increased by combining with a single substitution of a selected residue.

### Example 4: Enantioselectivity

Hydrolysis reactions were performed in 2 mL scale using 2 mM pNP 2-Me-butyrate as substrate in sodium phosphate buffer, 0.5 M pH 7.0 with 1% Triton X-100. The reactions were stopped by addition of 2 M HCl (0.1 mL), and then extracted into Et₂O (2 mL). After analysis by chiral GC (Varian CP-Chiralsil-DEX CB 10 m colum, temperature program 80 to 180 °C at 2 °C/min), E (enantiomeric ratio) was calculated as E = In[eeₚ(1-eeₛ)/(eeₚ+eeₛ)] / In[eeₚ(1+eeₛ)/(eeₚ+eeₛ)], with eeₛ and eeₚ being ee (enantiomeric excess) for substrate and product, respectively. Reactions were performed in triplets for each enzyme (stopped at different conversions) and E reported as an average.

CALB was tested and compared with variant Y135F, K136H, V139M, G142Y, P143G, D145C, L147G, A148N, V149F, S150GKVAKAGAPC, A151P, W155L. The results were E=2.4 for the variant and E=1.05 for the parent lipase (CALB), showing that CALB is almost entirely non-selective, but the variant has an increased enantioselectivity.

### Example 5: Hydrolysis of long-chain fatty acid ester

Michaelis-Menten constants were determined for a CALB variant with pNP laurate as a long-chain substrate. Experiments were performed in 0.5 M sodium phosphate buffer, pH 7.0, containing 1% Triton X-100 (to avoid turbid solutions at high substrate concentrations).

| | **k_{cat} (s⁻¹)** | **K_{M} (micro-M)** | **k_{cat} / K_{M} (s⁻¹ M⁻¹)** |
|---|---|---|---|
| Parent (caLB) | 3.1 | 535 | 0.58 * 10⁴ |
| V139I, G142N, P143I, L144G, D145G, L147T, A148G, V149L, S150IN, A151T, S153A, W155V | 23 | 170 | 14 * 10⁴ |

The results show that the variant is 23 times more active than the parent lipase on the long-chain substrate (measured as k_{cat} / K_{M}).

### Example 6: Hydrolysis of iso-propyl ester

The variant used in the previous example was also tested in hydrolysis of iso-propyl palmitate. The results showed that the hydrolysis was 26 % higher for the variant than for CALB. The hydrolysis was performed as follows:

As substrate, isopropylpalmitate was added to a concentration of 3 mg/ml in 50 mM NaAcetate pH 5.0 (= buffer), heated to 60°C for 5 minutes and homogenized by Ultra Turrax for 45 seconds and used immediately after preparation. Purified enzyme preparations were diluted to a concentration corresponding to OD280=0.00016 in desalted water and 10 ppm Triton X-100. In PCR-plates 20 micro-L buffer, 60 micro-L substrate and 20 micro-L enzyme solution were mixed at 800 RPM for 20 seconds and transferred to a PCR thermocycler for 30 minutes reaction at 30 C followed by 5 minutes at 90°C to inactivate enzymes and addition of 20 micro-L 10% solution of TritonX100 (in desalted water). The amount for fatty acids produced was determined using the NEFA C kit from Wako and results were calculated as an average of 6 determinations and subtraction of enzyme blank.

### Example 7: Activity at high pH

Lipase activity of two CALB variants was measured at various pH at 30°C with tributyrin as substrate and gum arabic as emulsifier. The results are expressed as relative activity, taking activity at pH 7.0 as 100.

| | pH 5.0 | pH 6.0 | pH 7.0 | pH 8.0 | pH 9.0 |
|---|---|---|---|---|---|
| Y135F, K136H, V139M, G142Y, P143G, D145C, L147G, A148N, V149F, S150GKVAKAGAPC, A151P, W155L | 53 | 97 | 100 | 90 | 151 |
| V139I, G142N, P143I, L144G, D145G, L147T, A148G, V149L, S150IN, A151T, S153A, W155V | 41 | 76 | 100 | 99 | 148 |
| Parent lipase (CALB) | 47 | 62 | 100 | 60 | 49 |

The variants are seen to have increased activity at alkaline pH (pH 7-9) and a higher pH optimum.

### Example 8: Synthesis reactions

The variants were immobilized on Accurel porous polypropylene by physical adsorption to a loading of 20 mg/g (based on A280). Reactions were performed in Eppendorf tubes with 1 mmol of each reagent, approx. 0.8 mL hexane, and 5 mg immobilized enzyme @ 40 °C, 1200 rpm. Samples were withdrawn for analysis by NMR and chiral GC.

Results from a synthesis reaction with 2-ethyl-1-hexanol and vinyl acetate as reactants are shown below as conversion % (ee %):

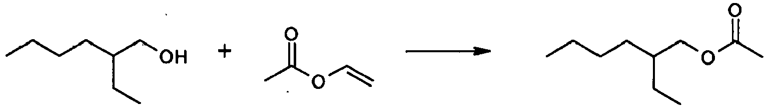

| **Variant** | 15 min | 30 min | 1 h | 2h | 3 h |
|---|---|---|---|---|---|
| Parent (CaLB) | 11 (32) | 24 (27) | 43 (22) | 61 (17) | 63 (17) |
| Y135F K136H V139M G142Y P143G D145C L147G A148N V149F S150GKVAKAGAPC A151P W155L | 6 (46) | 13 (46) | 24 (45) | 41 (40) | 52 (38) |
| V139I G142N P143I L144G D145G L147T A148G V149L S150IN A151T S153A W155V | 0.1 (51) | 6 (49) | 12 (50) | 22 (49) | 33 (48) |

The enantiomeric ratio was calculated by the formula given above. The results were E=1.9 for the parent lipase (CALB), and E=3.0 and E=3.2 for the two variants. Thus, the results show improved enantioselectivity for the two variants.

Another experiment was made in the same manner, but with vinyl benzoate and 1-hexanol as reactants.

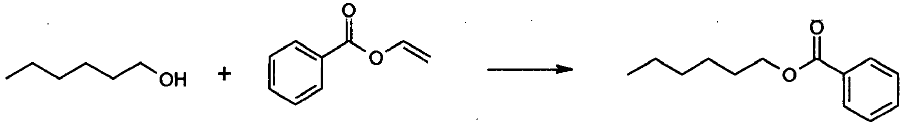

After 72 hours, a conversion of 17 % was found for the variant 1285E, whereas the parent CALB gave 9 %.

## Claims

1. A polypeptide which:
a) has lipolytic enzyme activity, and
b) has an amino acid sequence which has at least 80 % identity to SEQ ID NO: 1 and compared to SEQ ID NO: 1 comprises an amino acid substitution corresponding to D223G.

2. The polypeptide of claim 1 in immobilized form.

3. A method of performing a lipase-catalyzed reaction, which comprises contacting reactants with the polypeptide of any of claims 1 or 2, wherein the reaction is:
a) hydrolysis with a carboxylic acid ester and water as reactants, and a free carboxylic acid and an alcohol as products,
b) ester synthesis with a free carboxylic acid and an alcohol as reactants, and a carboxylic acid ester as product,
c) alcoholysis with a carboxylic acid ester and an alcohol as reactants, or
d) acidolysis with a carboxylic acid ester and a free fatty acid as reactants.

4. The method of claim 3, wherein the reaction is hydrolysis of an iso-propyl ester, or ester synthesis or alcoholysis with iso-propanol as a reactant.

## Patentansprüche

1. Polypeptid, das:
a) lipolytische Enzymaktivität aufweist, und
b) eine Aminosäuresequenz aufweist, die mindestens 80% Identität mit SEQ ID NO: 1 aufweist und verglichen mit SEQ ID NO: 1 eine Aminosäuresubstitution umfasst, die D223G entspricht.

2. Polypeptid nach Anspruch 1 in immobilisierter Form.

3. Verfahren zum Durchführen einer lipasekatalysierten Reaktion, das In-Kontakt-Bringen von Reaktanden mit dem Polypeptid gemäß einem beliebigen der Ansprüche 1 oder 2 umfasst, wobei die Reaktion ist:
a) Hydrolyse mit einem Carbonsäureester und Wasser als Reaktanden, und einer freien Carbonsäure und einem Alkohol als Produkte,
b) Estersynthese mit einer freien Carbonsäure und einem Alkohol als Reaktanden, und einem Carbonsäureester als Produkt,
c) Alkoholyse mit einem Carbonsäureester und einem Alkohol als Reaktanden, oder
d) Azidolyse mit einem Carbonsäureester und einer freien Fettsäure als Reaktanden.

4. Verfahren nach Anspruch 3, wobei die Reaktion Hydrolyse eines Isopropylesters, oder Estersynthese oder Alkoholyse mit Isopropanol als einem Reaktanden ist.

## Revendications

1. Un polypeptide qui :
a) a une activité enzymatique lipolytique, et
b) a une séquence d'acides aminés qui a au moins 80% d'identité avec la SEQ ID NO : 1 et, comparée à la SEQ ID NO : 1, comprend une substitution d'acide aminé correspondant à D223G.

2. Le polypeptide de la revendication 1 sous une forme immobilisée.

3. Un procédé pour effectuer une réaction catalysée par une lipase, qui comprend la mise en contact des réactifs avec le polypeptide de la revendication 1 ou 2, dans lequel la réaction est :
a) une hydrolyse avec un ester d'acide carboxylique et de l'eau comme réactifs, et un acide carboxylique libre et un alcool comme produits,
b) une synthèse d'ester avec un acide carboxylique libre et un alcool comme réactifs, et un ester d'acide carboxylique comme produit,
c) une alcoolyse avec un ester d'acide carboxylique et un alcool comme réactifs, ou
d) une acidolyse avec un ester d'acide carboxylique et un acide gras libre comme réactifs.

4. Le procédé de la revendication 3, dans lequel la réaction est une hydrolyse d'un ester d'iso-propyle, ou une synthèse d'ester ou une alcoolyse avec l'iso-propanol comme réactif.


### SEQUENCE LISTING

<110> Novozymes A/S
<120> Lipolytic Enzyme Variants
<130> 10617-WO
<160> 8
<170> PatentIn version 3.4
<210> 1
   <211> 317
   <212> PRT
   <213> Candida antarctica
<400> 1
<210> 2
   <211> 319
   <212> PRT
   <213> Hyphozyma sp.
<400> 2
   Phe Thr Pro Phe Pro Thr Gly Ala Asp Pro Ala Phe Thr Gln Ser Gln
   1               5                   10                  15
   Ala Thr Leu Asp Ala Gly Leu Thr Cys Gln Ser Gly Ser Pro Ser Ser
               20                  25                  30
   Gln Lys Asn Pro Ile Leu Leu Val Pro Gly Thr Gly Asn Thr Gly Pro
           35                  40                  45
   Gln Ser Phe Asp Ser Asn Trp Ile Pro Leu Ser Ala Gln Leu Gly Tyr
       50                  55                  60
   Ser Pro Cys Trp Val Ser Pro Pro Pro Phe Met Leu Asn Asp Ser Gln
   65                  70                  75                  80
   Ile Asn Ala Glu Tyr Ile Val Asn Ala Ile His Thr Leu Ser Ser Gly
                   85                  90                  95
   Ser Gly Ser Lys Val Pro Val Leu Thr Trp Ser Gln Gly Gly Leu Ala
               100                 105                 110
   Ala Gln Trp Ala Leu Thr Phe Phe Pro Ser Thr Arg Asn Lys Val Asp
           115                 120                 125
   Arg Leu Met Ala Phe Ala Pro Asp Tyr Lys Gly Thr Val Glu Ala Gly
       130                 135                 140
   Leu Leu Asp Ala Phe Gly Leu Ser Ala Pro Ser Val Trp Gln Gln Thr
   145                 150                 155                 160
   Ala Gln Ser Ala Phe Val Thr Ala Leu Asp Gln Ala Gly Gly Leu Asn
                   165                 170                 175
   Gln Ile Val Pro Thr Thr Asn Leu Tyr Ser Ala Thr Asp Glu Val Val
               180                 185                 190
   Gln Pro Gln Phe Ala Asn Gly Pro Pro Asp Ser Ser Tyr Leu Ser Asn
           195                 200                 205
   Gly Lys Asn Ile Gln Ala Gln Ser Ile Cys Gly Pro Leu Phe Ile Ile
       210                 215                 220
   Gly His Ala Gly Ser Leu Tyr Ser Gln Phe Ser Tyr Val Val Gly Lys
   225                 230                 235                 240
   Ser Ala Leu Ala Ser Pro Thr Gly Gln Ala Gln Ser Ser Asp Tyr Ser
                   245                 250                 255
   Ile Lys Asp Cys Asn Pro Ala Pro Ala Asn Pro Leu Thr Ala Gln Gln
   260 265 270
   Lys Leu Asp Ser Ala Ala Ile Ile Leu Val Ala Gly Lys Asn Ile Val
   275 280 285
   Thr Gly Pro Lys Gln Asn Cys Glu Pro Asp Leu Met Pro Tyr Ala Arg
   290 295 300
   Lys Tyr Arg Ile Gly Lys Lys Thr Cys Ser Gly Val Ile Thr Gly
   305 310 315
<210> 3
   <211> 336
   <212> PRT
   <213> Ustilago maydis
<400> 3
   Met Lys Thr Thr Ser Val Ile Ser Ala Leu Val Thr Leu Ala Ser Ile
   1 5 10 15
   Ile Arg Ala Ala Pro Leu Ala Ser Ser Asp Pro Ala Phe Ser Thr Pro
   20 25 30
   Lys Ala Thr Leu Asp Ala Gly Leu Glu Cys Gln Thr Gly Ser Pro Ser
   35 40 45
   Ser Gln Thr Lys Pro Ile Leu Leu Val Pro Gly Thr Gly Ala Asn Gly
   50 55 60
   Thr Gln Thr Phe Asp Ser Ser Trp Ile Pro Leu Ser Ala Lys Leu Gly
   65 70 75 80
   Phe Ser Pro Cys Trp Ile Ser Pro Pro Pro Phe Met Leu Asn Asp Ser
   85 90 95
   Gln Val Asn Val Glu Tyr Ile Val Asn Ala Val Gln Thr Leu Tyr Ala
   100 105 110
   Gly Ser Gly Ser Lys Lys Val Pro Val Leu Thr Trp Ser Gln Gly Gly
   115 120 125
   Leu Ala Thr Gln Trp Ala Leu Thr Phe Phe Pro Ser Ile Arg Asn Gln
       130                 135                 140
   Val Asp Arg Leu Met Ala Phe Ala Pro Asp Tyr Lys Gly Thr Ile Glu
   145                 150                 155                 160
   Ala Gly Leu Leu Ser Thr Phe Gly Leu Ala Ser Gln Ser Val Trp Gln
                   165                 170                 175
   Gln Gln Ala Gly Ser Ala Phe Val Thr Ala Leu Lys Asn Ala Gly Gly
               180                 185                 190
   Leu Thr Ser Phe Val Pro Thr Thr Asn Leu Tyr Ser Phe Phe Asp Glu
           195                 200                 205
   Ile Val Gln Pro Gln Val Phe Asn Ser Asp Ala Asp Ser Ser Tyr Leu
       210                 215                 220
   Gly Asn Ser Lys Asn Ile Gln Ala Gln Thr Val Cys Gly Gly Phe Phe
   225                 230                 235                 240
   Val Ile Asp His Ala Gly Ser Leu Thr Ser Gln Phe Ser Tyr Val Val
                   245                 250                 255
   Gly Lys Ser Ala Leu Thr Ser Ser Ser Gly Val Ala Asn Ser Ala Asp
               260                 265                 270
   Tyr Ser Ser Lys Asp Cys Lys Ala Ser Pro Ala Asp Asp Leu Ser Ala
           275                 280                 285
   Lys Gln Lys Ala Asp Ala Ser Ala Leu Leu Phe Val Ala Ala Gly Asn
       290                 295                 300
   Leu Leu Ala Gly Pro Lys Gln Asn Cys Glu Pro Asp Leu Lys Pro Tyr
   305                 310                 315                 320
   Ala Arg Gln Phe Ala Val Gly Lys Lys Thr Cys Ser Gly Thr Ile Asn
                   325                 330                 335
<210> 4
   <211> 445
   <212> PRT
   <213> Gibberella zeae
<400> 4
   Ala Pro Ser Tyr Ser Asp Leu Glu Ser Arg Gln Leu Ile Gly Gly Leu
   1               5                   10                  15
   Leu Lys Gly Val Asp Gly Thr Leu Glu Thr Val Val Gly Gly Leu Leu
               20                  25                  30
   Gly Thr Leu Arg Lys Ala Ile Asp Ser Gly Asp Arg Asp Lys Thr Leu
           35                  40                  45
   Asp Ile Leu His Val Leu Glu Pro Ala Lys Lys His Lys Asn Val Glu
       50                  55                  60
   Glu Ala Phe Ala Ala Leu Glu Lys Ile Ser Lys Ser Lys Pro Lys Thr
   65                  70                  75                  80
   Ile Ile Asp Tyr Ser Ala Gln Leu Ile Val Asn Gly Leu Ile Ser Gly
                   85                  90                  95
   Asn Thr Leu Asp Leu Phe Ala Tyr Ala Lys Gly Leu Val Ser Ala Gln
               100                 105                 110
   Asn Gly Ser Asn Asn Lys Asn Arg Asn Pro Pro Lys Glu Val Tyr Pro
           115                 120                 125
   Lys Val Ala Asn Cys Asp Ala Ser Tyr Thr Thr Ser Glu Ala Lys Leu
       130                 135                 140
   Arg Ala Ala Ile His Ile Pro Pro Thr Phe Thr Tyr Gly Glu Lys Pro
   145                 150                 155                 160
   Pro Val Ile Leu Phe Pro Gly Thr Gly Ser Thr Gly Phe Thr Thr Tyr
                   165                 170                 175
   Arg Gly Asn Phe Ile Pro Leu Leu Thr Asp Val Glu Trp Ala Asp Pro
               180                 185                 190
   Val Trp Val Asn Val Pro Val Leu Leu Leu Glu Asp Ala Gln Val Asn
           195                 200                 205
   Ala Glu Tyr Ala Ala Tyr Ala Leu Asn Tyr Ile Ala Ser Leu Thr Lys
       210                 215                 220
   Arg Asn Val Ser Val Ile Ala Trp Ser Gln Gly Asn Ile Asp Val Gln
   225                 230                 235                 240
   Trp Ala Leu Lys Tyr Trp Pro Ser Thr Arg Lys Val Thr Thr Asp His
                   245                 250                 255
   Val Ala Ile Ser Ala Asp Tyr Lys Gly Thr Ile Leu Ala Asn Ile Gly
               260                 265                 270
   Gly Ala Thr Gly Leu Ile Asn Thr Pro Ala Val Val Gln Gln Glu Ala
           275                 280                 285
   Gly Ser Thr Phe Ile Asn Thr Leu Arg Ser Asn Asp Gly Asp Ser Gly
       290                 295                 300
   Tyr Ile Pro Thr Thr Ser Leu Tyr Ser Ser Leu Phe Asp Glu Val Val
   305                 310                 315                 320
   Gln Pro Gln Glu Gly Ala Gly Ala Ser Ala Tyr Leu Leu Asp Ala Arg
                   325                 330                 335
   Asp Val Gly Val Thr Asn Ala Glu Val Gln Lys Val Cys Thr Gly Lys
               340                 345                 350
   Leu Gly Gly Ser Phe Tyr Thr His Glu Ser Met Leu Ala Asn Pro Leu
           355                 360                 365
   Thr Phe Ala Leu Ala Lys Asp Ala Leu Thr His Glu Gly Pro Gly Thr
       370                 375                 380
   Ile Ser Arg Leu Asp Leu Ala Asp Val Cys Asn Arg Ser Leu Ala Pro
   385                 390                 395                 400
   Gly Leu Gly Leu Lys Asp Leu Leu Ile Thr Glu Asn Ala Val Val Ile
                   405                 410                 415
   Ala Ala Leu Ser Leu Val Leu Tyr Leu Pro Lys Gln Ile Asp Glu Pro
               420                 425                 430
   Ala Ile Lys Gln Tyr Ala Leu Glu Ala Thr Gly Thr Cys
               435                 440                 445
<210> 5
   <211> 455
   <212> PRT
   <213> Debaryomyces hansenii
<400> 5
   His Pro Thr Lys Glu Leu Glu Arg Arg Asp Leu Ile Ser Asn Ile Asp
   1               5                   10                  15
   Asp Ile Val Asn Ser Thr Ile Asp Asn Gly Glu Ala His Lys Asp Asn
               20                  25                  30
   Ala Lys Ser Ala Ile Thr Asp Ile Phe Asp Lys Ile Asn Asp Gly Ile
           35                  40                  45
   Lys Gln Asp Ile Asp Asn Leu Lys Glu Val Gly Lys Ser Ile Ala Asp
       50                  55                  60
   Leu Ile Lys Ser Val Val Pro Thr Glu Asp Leu Ser Thr Pro Glu Gly
   65                  70                  75                  80
   Val Gln Ala Tyr Leu Gly Gln Leu Phe Glu Asn Gly Glu Asp Leu Phe
                   85                  90                  95
   Lys Asn Ser Ile Asp Met Val Gly His Gly Leu Lys Pro Gly Ser Ile
               100                 105                 110
   Ala Gly Asn Phe Glu Gly Phe Ser Asp Glu Ile Asn Thr Ser Asp Asn
           115                 120                 125
   Phe Asn Val Lys Glu Pro Glu Gly Ser Val Tyr Pro Gln Ala Glu Ser
       130                 135                 140
   Glu Asp Pro Ser Phe Ser Leu Ser Glu Glu Gln Leu Arg Ser Ala Ile
   145                 150                 155                 160
   Gln Ile Pro Glu Glu Phe Gln Tyr Gly Asn Gly Ser Lys Ser Pro Val
                   165                 170                 175
   Ile Leu Val Pro Gly Thr Gly Ser Lys Gly Gly Met Thr Tyr Ala Ser
               180                 185                 190
   Asn Tyr Ala Lys Leu Leu Lys Glu Thr Asp Phe Ala Asp Val Val Trp
           195                 200                 205
   Leu Asn Val Pro Gly Tyr Leu Leu Asp Asp Ala Gln Asn Asn Ala Glu
       210                 215                 220
   Tyr Val Ala Tyr Ala Ile Asn Tyr Ile Ser Gly Ile Ser Asn Asn Lys
   225                 230                 235                 240
   Asn Val Ser Ile Ile Ser Trp Ser Gln Gly Gly Leu Asp Thr Gln Trp
                   245                 250                 255
   Ala Leu Lys Tyr Trp Ala Ser Thr Arg Ser Lys Val Ser Asp Phe Ile
               260                 265                 270
   Pro Ile Ser Pro Asp Phe Lys Gly Thr Arg Met Val Pro Val Leu Cys
           275                 280                 285
   Pro Ser Phe Pro Lys Leu Ser Cys Pro Pro Ser Val Leu Gln Gln Glu
       290                 295                 300
   Tyr Asn Ser Thr Phe Ile Glu Thr Leu Arg Ala Asp Gly Gly Asp Ser
   305                 310                 315                 320
   Ala Tyr Val Pro Thr Thr Ser Ile Tyr Ser Gly Phe Asp Glu Ile Val
                   325                 330                 335
   Gln Pro Gln Ser Gly Lys Gly Ala Ser Gly Leu Ile Asn Asp Asn Arg
               340                 345                 350
   Asn Val Gly Val Thr Asn Asn Glu Val Gln Thr Ile Cys Pro Asp Arg
           355                 360                 365
   Pro Ala Gly Lys Tyr Tyr Thr His Glu Gly Val Leu Tyr Asn Pro Val
       370                 375                 380
   Gly Tyr Ala Leu Ala Val Asp Ala Leu Thr His Glu Gly Pro Gly Gln
   385                 390                 395                 400
   Leu Ser Arg Ile Asp Leu Asp Thr Glu Cys Gly Arg Ile Val Pro Asp
   405 410 415
   Gly Leu Thr Tyr Thr Asp Leu Leu Ala Thr Glu Ala Leu Ile Pro Glu
   420 425 430
   Ala Leu Val Leu Ile Leu Ser Tyr Asp Asp Lys Thr Arg Asp Glu Pro
   435 440 445
   Glu Ile Arg Ser Tyr Ala Gln
   450 455
<210> 6
   <211> 440
   <212> PRT
   <213> Aspergillus fumigatus
<400> 6
   Ala Val Ile Pro Arg Gly Ala Val Pro Val Ala Ser Asp Leu Ser Leu
   1 5 10 15
   Val Ser Ile Leu Ser Ser Ala Ala Asn Asp Ser Ser Ile Glu Ser Glu
   20 25 30
   Ala Arg Ser Ile Ala Ser Leu Ile Ala Ser Glu Ile Val Ser Lys Ile
   35 40 45
   Gly Lys Thr Glu Phe Ser Arg Ser Thr Lys Asp Ala Lys Ser Val Gln
   50 55 60
   Glu Ala Phe Asp Lys Ile Gln Ser Ile Phe Ala Asp Gly Thr Pro Asp
   65 70 75 80
   Phe Leu Lys Met Thr Arg Glu Ile Leu Thr Val Gly Leu Ile Pro Ala
   85 90 95
   Asp Ile Val Ser Phe Leu Asn Gly Tyr Leu Asn Leu Asp Leu Asn Ser
   100 105 110
   Ile His Asn Arg Asn Pro Ser Pro Lys Gly Gln Ala Ile Tyr Pro Val
   115 120 125
   Lys Ala Pro Gly Asp Ala Arg Tyr Ser Val Ala Glu Asn Ala Leu Arg
   130 135 140
   Ala Ala Ile His Ile Pro Ala Ser Phe Gly Tyr Gly Lys Asn Gly Lys
   145                 150                 155                 160
   Lys Pro Val Ile Leu Val Pro Gly Thr Ala Thr Pro Ala Gly Thr Thr
                   165                 170                 175
   Tyr Tyr Phe Asn Phe Gly Lys Leu Gly Ser Ala Ala Asp Ala Asp Val
               180                 185                 190
   Val Trp Leu Asn Ile Pro Gln Ala Ser Leu Asn Asp Val Gln Ile Asn
           195                 200                 205
   Ser Glu Tyr Val Ala Tyr Ala Ile Asn Tyr Ile Ser Ala Ile Ser Glu
       210                 215                 220
   Ser Asn Val Ala Val Leu Ser Trp Ser Gln Gly Gly Leu Asp Thr Gln
   225                 230                 235                 240
   Trp Ala Leu Lys Tyr Trp Pro Ser Thr Arg Lys Val Val Asp Asp Phe
                   245                 250                 255
   Ile Ala Ile Ser Pro Asp Phe His Gly Thr Val Met Arg Ser Leu Val
               260                 265                 270
   Cys Pro Trp Leu Ala Ala Leu Ala Cys Thr Pro Ser Leu Trp Gln Gln
           275                 280                 285
   Gly Trp Asn Thr Glu Phe Ile Arg Thr Leu Arg Gly Gly Gly Gly Asp
       290                 295                 300
   Ser Ala Tyr Val Pro Thr Thr Thr Ile Tyr Ser Thr Phe Asp Glu Ile
   305                 310                 315                 320
   Val Gln Pro Met Ser Gly Ser Gln Ala Ser Ala Ile Leu Ser Asp Ser
                   325                 330                 335
   Arg Ala Val Gly Val Ser Asn Asn His Leu Gln Thr Ile Cys Gly Gly
               340                 345                 350
   Lys Pro Ala Gly Gly Val Tyr Thr His Glu Gly Val Leu Tyr Asn Pro
           355                 360                 365
   Leu Ala Trp Ala Leu Ala Val Asp Ala Leu Ser His Asp Gly Pro Gly
   370 375 380
   Asp Pro Ser Arg Leu Asp Leu Asp Val Val Cys Gly Arg Val Leu Pro
   385 390 395 400
   Pro Gln Leu Gly Leu Asp Asp Leu Leu Gly Thr Glu Gly Leu Leu Leu
   405 410 415
   Ile Ala Leu Ala Glu Val Leu Ala Tyr Lys Pro Lys Thr Phe Gly Glu
   420 425 430
   Pro Ala Ile Ala Ser Tyr Ala His
   435 440
<210> 7
   <211> 401
   <212> PRT
   <213> Aspergillus oryzae
<400> 7
   Leu Pro Ser Ser Ser Glu Thr Val Glu Ala Asn Cys Val Lys Pro Tyr
   1 5 10 15
   Leu Cys Cys Gly Glu Leu Lys Thr Pro Leu Asp Ser Thr Leu Asp Pro
   20 25 30
   Ile Leu Leu Asp Leu Gly Ile Asp Ala Ala Ser Ile Val Gly Ser Val
   35 40 45
   Gly Leu Leu Cys Leu Ile Pro Ser Lys Ala Leu Thr Cys Leu Asn Gly
   50 55 60
   Tyr Ala Ile Ile Asp Leu Asn Ser Ile His Arg His Asn Pro Ser Pro
   65 70 75 80
   Glu Asn Leu Ser Ile Tyr Pro Tyr Lys Ala Lys Ser Asp Ala Pro Tyr
   85 90 95
   Ser Ile Ala Glu Asn Thr Leu Arg Ala Ala Ile His Ile Pro Arg Ser
   100 105 110
   Phe Ser His Lys Arg Asp Lys Lys Ile Pro Val Leu Leu Val Pro Gly
           115                 120                 125
   Thr Ala Val Pro Ala Ala Ile Thr Phe Tyr Phe Asn Phe Gly Lys Leu
       130                 135                 140
   Arg Arg Ala Leu Pro Glu Ser Glu Leu Val Trp Ile Asp Leu Pro Gln
   145                 150                 155                 160
   Ala Ser Leu Asp Asp Ile Gln Leu Ser Ala Glu Tyr Val Ala Tyr Ala
                   165                 170                 175
   Leu Asn Tyr Val Ser Ala Leu Thr Ser Ser Lys Ile Ala Val Ile Ser
               180                 185                 190
   Trp Ser Gln Gly Ala Leu Asp Ile Gln Trp Ala Leu Lys Tyr Trp Pro
           195                 200                 205
   Ser Thr Arg Ser Val Val Asn Asp Phe Ile Ala Ile Ser Pro Asp Phe
       210                 215                 220
   His Gly Thr Ile Val Lys Trp Leu Val Cys Pro Leu Leu Asn Asp Leu
   225                 230                 235                 240
   Ala Cys Thr Pro Ser Ile Trp Gln Gln Gly Trp Asp Ala Asn Phe Ile
                   245                 250                 255
   Gln Ala Leu Arg Ser Gln Gly Gly Asp Ser Ala Tyr Val Thr Thr Thr
               260                 265                 270
   Thr Ile Tyr Ser Ser Phe Asp Lys Ile Val Arg Pro Met Ser Gly Glu
           275                 280                 285
   Asn Ala Ser Ala Arg Leu Leu Asp Tyr Arg Gly Val Gly Val Ser Asn
       290                 295                 300
   Asn His Leu Gln Thr Ile Cys Ala Asn Asn Ala Ala Gly Gly Leu Tyr
   305                 310                 315                 320
   Thr His Glu Gly Val Leu Tyr Asn Pro Leu Ala Trp Ala Leu Thr Val
                   325                 330                 335
   Asp Ala Leu Leu His Asp Gly Pro Ser Asn Ile Thr Arg Ile Asp Thr
   340 345 350
   Gln Lys Ile Cys Glu Gln Val Leu Pro Pro Tyr Leu Glu Leu Thr Asp
   355 360 365
   Met Leu Gly Thr Glu Ala Leu Leu Leu Val Ala Leu Ala Lys Ile Leu
   370 375 380
   Thr Tyr Ser Pro Lys Val Ser Gly Glu Pro Asp Ile Ala Lys Tyr Ala
   385 390 395 400
   Tyr
<210> 8
   <211> 388
   <212> PRT
   <213> Neurospora crassa
<400> 8
   Leu Pro Thr Thr Ser Glu Pro Val His His Glu Ser Val Arg Ala Ile
   1 5 10 15
   Gly Glu Leu Ser His Arg Asp Glu Leu His Asp Ala Gly Val Val Trp
   20 25 30
   Asn Lys Val Val Arg Gln Ser Pro Leu Val Ala Pro Thr Asp Pro Arg
   35 40 45
   Asp Ser Phe Asn Asn Gln Asn Pro Asp Val Pro Gly Val Gly Tyr Pro
   50 55 60
   Arg Ser Ser Asp Ala Asp Pro Ala Phe Thr Ile Pro Glu Ala Lys Leu
   65 70 75 80
   Arg Ser Ala Ile Tyr Leu Pro Ser Gly Phe Asn Ser Ser Thr Asn Arg
   85 90 95
   Gln Val Val Leu Phe Val Pro Gly Thr Gly Ala Tyr Gly His Glu Ser
   100 105 110
   Phe Ala Asp Asn Leu Leu Lys Val Ile Thr Asn Ala Gly Ala Ala Asp
           115                 120                 125
   Ala Val Trp Val Asn Val Pro Asn Ala Met Leu Asp Asp Val Gln Ser
       130                 135                 140
   Asn Ala Glu Tyr Ile Ala Tyr Ala Ile Ser Tyr Val Lys Ala Leu Ile
   145                 150                 155                 160
   Gly Asp Asp Arg Asp Leu Asn Val Ile Gly Trp Ser Gln Gly Asn Leu
                   165                 170                 175
   Ala Thr Gln Trp Val Leu Thr Tyr Trp Pro Ser Thr Ala Pro Lys Val
               180                 185                 190
   Arg Gln Leu Ile Ser Val Ser Pro Asp Phe His Gly Thr Met Leu Ala
           195                 200                 205
   Tyr Gly Leu Cys Ala Gly Asn Phe Gly Lys Val Ala Lys Ala Gly Ala
       210                 215                 220
   Pro Cys Pro Pro Ser Val Leu Gln Gln Leu Tyr Ser Ser Asn Leu Ile
   225                 230                 235                 240
   Asn Thr Leu Arg Ala Ala Gly Gly Gly Asp Ala Gln Val Pro Thr Thr
                   245                 250                 255
   Ser Phe Trp Ser Arg Leu Thr Asp Glu Val Val Gln Pro Gln Ala Gly
               260                 265                 270
   Leu Thr Ala Ser Ala Arg Met Gly Asp Ala Arg Asn Lys Gly Val Thr
           275                 280                 285
   Asn Val Glu Val Gln Thr Val Cys Gly Leu Ser Val Gly Gly Gly Gln
       290                 295                 300
   Tyr Gly His Ser Thr Leu Met Ala His Pro Leu Val Ala Ala Met Thr
   305                 310                 315                 320
   Leu Asp Ala Leu Lys Asn Gly Gly Pro Ala Ser Leu Ser Arg Ile Arg
                   325                 330                 335
   Ser Gln Met Phe Arg Ala Cys Ser Asn Val Val Ala Pro Gly Leu Gln
               340                 345                     350
   Leu Thr Asp Arg Ala Lys Thr Glu Gly Leu Leu Ala Thr Ala Gly Ala
           355                 360                     365
   Arg Met Gly Ala Phe Pro Thr Lys Leu Leu Arg Glu Pro Ala Leu Arg
       370                 375                     380
   Gln Tyr Ala Ala
   385